(19) Europäisches Patentamt
European Patent Office
Office européen des brevets

(11) **EP 3 601 520 B1**

(12) **EUROPEAN PATENT SPECIFICATION**

(45) Date of publication and mention
of the grant of the patent:
**26.05.2021 Bulletin 2021/21**

(21) Application number: **18713197.4**

(22) Date of filing: **21.03.2018**

(51) Int Cl.:
**C12M 1/12** (2006.01)          **C12M 1/34** (2006.01)
**G01N 33/18** (2006.01)

(86) International application number:
**PCT/EP2018/057203**

(87) International publication number:
**WO 2018/172424 (27.09.2018 Gazette 2018/39)**

(54) **SENSOR DEVICE FOR TRACKING POSITION AND PROCESS PARAMETERS IN A CONTAINER**

SENSORVORRICHTUNG ZUR VERFOLGUNG VON POSITION UND PROZESSPARAMETERN IN EINEM BEHÄLTER

DISPOSITIF CAPTEUR POUR SUIVRE LA POSITION ET LES PARAMÈTRES DE PROCESSUS DANS UN CONTENEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.03.2017 EP 17162273**

(43) Date of publication of application:
**05.02.2020 Bulletin 2020/06**

(73) Proprietor: **Freesense ApS
2100 Copenhagen Ø (DK)**

(72) Inventors:
• **VAEVER PETERSEN, Lars
2000 Copenhagen N (DK)**
• **SKYGGEBJERG, Ole
2000 Copenhagen N (DK)**
• **NØRREGAARD, Anders
2000 Copenhagen N (DK)**
• **JACOBSEN, Jesper Bryde
2000 Copenhagen N (DK)**

(74) Representative: **ZBM Patents ApS
Symbion Box:33
Fruebjergvej 3
2100 Copenhagen Ø (DK)**

(56) References cited:
• **REINECKE SEBASTIAN FELIX ET AL:
"Macro-mixing characterisation of a stirred
model fermenter of non-Newtonian liquid by flow
following sensor particles and ERT", CHEMICAL
ENGINEERING RESEARCH AND DESIGN,
ELSEVIER, AMSTERDAM, NL, vol. 118, 9
December 2016 (2016-12-09), pages 1-11,
XP029898018, ISSN: 0263-8762, DOI:
10.1016/J.CHERD.2016.12.002 cited in the
application**
• **THIELE ET AL.: "Autonomous sensor particle for
parameter tracking in large vessels", MEAS. SCI.
TECHNOL., vol. 21, 2010, page 085201,
XP020195933, cited in the application**
• **DENG Z D ET AL: "Design and implementation of
a new autonomous sensor fish to support
advanced hydropower development", REVIEW
OF SCIENTIFIC INSTRUMENTS, AIP, MELVILLE,
NY, US, vol. 85, no. 11, 1 January 1901
(1901-01-01), XP012202281, ISSN: 0034-6748,
DOI: 10.1063/1.4900543 [retrieved on 1901-01-01]**
• **S. REINECKE ET AL: "Flow following sensor
particles-Validation and macro-mixing analysis
in a stirred fermentation vessel with a highly
viscous substrate", BIOCHEMICAL
ENGINEERING JOURNAL, vol. 69, 1 December
2012 (2012-12-01), pages 159-171, XP055409072,
NL ISSN: 1369-703X, DOI:
10.1016/j.bej.2012.09.010**

## Description

### Field of the invention

The present invention relates to a novel sensor device for tracking position and process parameters in a container (e.g. a bioreactor).

### Background of the invention

The acquisition of process parameters in large-scale vessels, such as large tanks, reactors, fermenters and bioreactors, is of great interest for the investigation and optimization of industrial processes. This is especially the case for bioreactors, where several process parameters, such as the temperature profile, distribution of pH, oxygen levels, growth rate of the biomass, gas-liquid fraction in the substrates as well as flow characteristics, such as velocity profiles, dead zone locations are of interest to operators.

Measurements of few basic physical parameters, such as temperature and local velocities, allows to draw conclusions regarding the efficiency of the mixing and heating regimes, but the acquisition of spatial parameter distributions in larger vessels is quite difficult. Moreover, the measurement and monitoring of significant parameters in reactor vessels is hampered by limited access to the process itself, because sensor mounting or cable connections are not feasible or desired. Thus, due to the large dimensions of many industrial vessels, the acquired data may not be fully representative of the whole volume.

The last years remote sensing and autonomous sensor concepts have gained increasing attention. For example, the article by Thiele et al 2010 describes a neutrally buoyant self-powered sensor particle for the measurement of process parameters including temperature, absolute pressure (i.e. immersion depth) and acceleration data.

In the Thiele article is determined the immersion depth and thereby the vertical position of the sensor device in the liquid medium of the container based the sampled pressure data wherein the pressure is correlated to the depth in the sense that higher pressure relates to increased immersion depth of the sensor device in the liquid medium of the container.

In the Thiele article is also determined one horizontal position of the sensor device in the liquid medium of the container based the sampled accelerometer data, wherein a characteristic acceleration profile is derived from the sensor device contacting the impeller/agitator. Further, the article by Reinecke et al., describes a particle sensor where the position determination is based on an electrical resistance tomography system (ERT) that was applied onto the bioreactor. Such a system is based on conductivity measurements and only allows for observation of the particle from the outside. Thus, in the article by Reinecke et al the detection of the particle is not based on acceleration, but were calculated from the temporal difference between the drops of the fluctuating conductivity signals from the ERT measurements.

### Summary of the invention

The problem to be solved by the present invention is to provide an improved sensor device for tracking process parameters (e.g. pH, temperature, etc.) in a container (e.g. bioreactors).

The solution is based on the finding by the present inventors that in a container (e.g. a bioreactor) comprising a liquid medium, a wall and an agitator (e.g. an impeller) it is possible to significantly improve in particular the precision in the determination of the horizontal position of the sensor device within the container based on the finding that it is possible to differentiate the acceleration profile derived from when the sensor device contacts the wall of the container from the acceleration profile derived from when the sensor device contacts the agitator.

Based on this finding, it is then possible to make a software algorithm capable of identifying this acceleration difference and thereby determining when the device contacts the agitator or contacts the wall of the container and thereby obtaining the position of the sensor device nearby the agitator or nearby the wall.

Accordingly, with respect to horizontal position the sensor device as described herein is capable of determining two different horizontal positions of the sensor device in the liquid medium of the container - i.e. when it contacts the wall and when it contacts the agitator. As discussed above, the sensor of the Thiele et al article only determines one horizontal position of the sensor device in the liquid medium of the container.

As discussed herein, the fact that the sensor device as described herein is capable of determining two different horizontal positions (i.e. when it contacts the wall and when it contacts the agitator) is a significant advantage in general. For instance, in relation to a preferred embodiment herein where the sensor devise also comprises a gyroscope sampling data about angular velocity one may get a significant improved determination in the precision of the position, orientation, and velocity (direction and speed of movement) of the moving sensor device within the liquid media, since one has two well defined previously determined positions (i.e. when contacting the wall or agitator) to use in a process of calculating the sensor device current position by using a previously determined position and the previously determined position is when the device contacts the agitator and when the device contacts the wall of the container.

In working example herein is shown an example of a sensor device of the present invention a process of calculating its position during different time periods of a fermentations reaction in a bioreactor container and measuring relevant fermentation reactions parameters such as temperature and pH levels.

Accordingly, a first aspect of the present invention relates to a sensor device comprising a house (10), wherein the house (10) comprises:

(a) a position determination unit (1) comprising an accelerometer and a pressure sensor,
(b) a microcontroller unit (3) comprising a software algorithm configured for determining position of the sensor device,
(c) a power management unit (5),
(d) a process parameter sensor (2), and
(e) a communication unit (4) adapted for communicating with a user interface; and

wherein the software algorithm in (b) can determine the position of the sensor device in a container comprising a liquid medium, a wall and an agitator wherein the algorithm is an algorithm comprising the following steps:

(i) sampling data about pressure and acceleration from the position determination unit (1) of item (a); and
(iia) determining the immersion depth and thereby the vertical position of the sensor device in the liquid medium of the container based on the in step (i) sampled pressure data wherein the pressure is correlated to the depth in the sense that higher pressure relates to increased immersion depth of the sensor device in the liquid medium of the container; and
(iib): determining horizontal position of the sensor device in the liquid medium of the container based on the in step (i) sampled accelerometer data, wherein a first acceleration profile is derived from the sensor device contacting the agitator and a second acceleration profile is derived from the sensor device contacting the wall of the container and wherein the first and seconds acceleration profiles are different and the algorithm is capable of identifying this difference and thereby determining when the device contacts the agitator or contacts the wall of the container.

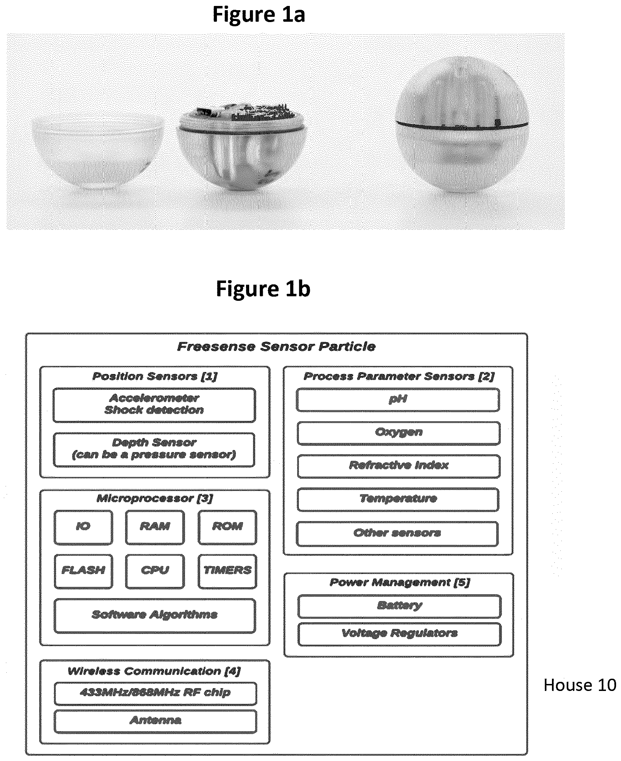

An example of a sensor device is shown in figure 1 - the non-limiting numbering in the first aspect refers to this figure 1.

The term "agitator" is well known in the art and is understood by the skilled person in the present context to relate to a rotating component that move liquid medium by rotation.
As known in the art - an example of a suitable agitator is an impeller.

The actual mathematics (e.g. integration based mathematics) and specific software language used for making a software algorithm of the first aspect may be seen as based on standard well known mathematics and software language - i.e. based on the teaching herein and the common general knowledge of the skilled person, it is routine work for the skilled person to make a software algorithm of the first aspect of the present invention.

A second aspect of the invention relates to use of the sensor device of first aspect and relevant embodiments thereof for measuring position and process parameters in a container comprising a liquid medium, a wall and an agitator.

A third aspect of the invention relates to a method for determining the position of the sensor device of the first aspect and any relevant embodiments thereof in a container comprising a liquid medium, a wall and an agitator comprising the steps of:

(i) sampling data about pressure and acceleration from the position determination unit (1) of item (a); and
(iia) determining the immersion depth and thereby the vertical position of the sensor device in the liquid medium of the container based on the in step (i) sampled pressure data wherein the pressure is correlated to the depth in the sense that higher pressure relates to increased immersion depth of the sensor device in the liquid medium of the container; and
(iib): determining horizontal position of the sensor device in the liquid medium of the container based on the in step (i) sampled accelerometer data, wherein a first acceleration profile is derived from the sensor device contacting the agitator and a second acceleration profile is derived from the sensor device contacting the wall of the container and wherein the first and seconds acceleration profiles are different and the algorithm is capable of identifying this difference and thereby determining when the device contacts the agitator or contacts the wall of the container.

Embodiments of the present invention are described below, by way of examples only.

**Drawing description**

3

Figure 1: An example of a sensor device of the present invention. In Figure 1a is shown an example of a sensor device in a two-part form - this sensor was used in Example 2 herein. In Figure 1b is shown a block diagram of the sensor device's electronics.

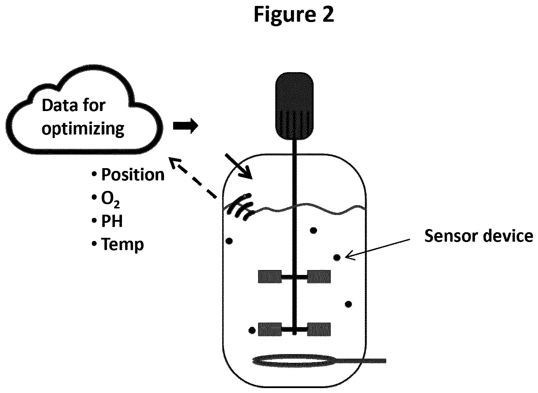

Figure 2: An example of a sensor device sampling process parameters in a bioreactor.

Figure 3: An example of a measured impact profile - see working Example 2 herein for further details.

Figure 4: Comparison of theoretically determined impact and experimentally determined impact - see working Example 2 herein for further details. □: Theoretically determined wall impact. o: Theoretically determined impeller impact. ■: Experimentally determined wall impact. ●: Experimentally determined impeller impact. Impacts outside the gray lines are a result of momentary pressure increases caused by impacts.

**Detailed description of the invention**

Sensor device:

The overall design of the sensor device is based on a house (10), wherein the house (10) comprises:

(a) a position determination unit (1) comprising an accelerometer and a pressure sensor,
(b) a microcontroller unit (3) comprising a software algorithm configured for determining position of the sensor device,
(c) a power management unit (5),
(d) a process parameter sensor (2), and
(e) a communication unit (4) adapted for communicating with a user interface;

An example of a sensor device is shown in figure 1.
Preferably, the weight of the sensor device is from 1 mg to 1 kg, such as from 1 g to 500 g or such as from 2 g to 250 g.
If the sensor device is shaped as a sphere - the size of the sensor device is a size with a diameter from 0,1 mm to 200 cm, such as a diameter from 1 mm to 100 cm or such as a diameter from 1 cm to 20 cm.

House (10)

The sensor device comprises a preferably rigid and stable, non-deformable house part that may be constructed from a bio compatible product including metal, wood, bamboo and plastic such as organic thermoplastic polymer plastic including polyether ether ketone (PEEK) and polyaryl ether ketone (PAEK). The house (10) may have one or more openings to the exterior thereby allowing the process parameter sensors (2) to obtain process date from the outside environment. In one embodiment, the sensor device is made of a bio compatible product including metal, wood, bamboo and plastic such as organic thermoplastic polymer plastic including polyether ether ketone (PEEK) and polyaryl ether ketone (PAEK).
The house (10) may be constructed as a two-part shell that can be opened and closed for adapting the type of process parameter sensors (2), the communication unit (4), power management unit (5) or other relevant components of the sensor device. It may however, also be constructed as a one peace closed body intended for single use usage. Thus, in one embodiment, the house (10) is on 2-part form. In another embodiment, the house (10) is molded as one.
The house (10) is normally constructed to be water and air tight for protecting the inside electronics from water and gasses. In one embodiment, the sensor device is water tight. In another embodiment, the sensor device is air tight.
The house (10) may be adapted to be neutrally buoyant, positively buoyant or negatively buoyant depending on the use of the sensor device. It may also be constructed to be a self-buoyant meaning that it is able to adjust its buoyancy to the depth of interest. In one embodiment, the sensor device is neutrally buoyant, positively buoyant or negatively buoyant. In a preferred embodiment, the sensor device is self-buoyant.
The house (10) holds the all the necessary elements required for the sensor device to operate and collect the relevant process parameters. Such elements include but are not limited to
a position determination unit (1), a microcontroller unit (3), a power management unit (5), a process parameter sensor (2), a communication unit (4). In one embodiment, the house (10) further comprises a data storage unit.

Position determination unit (1)

The position determination unit (1) comprises an accelerometer and a pressure sensor.

Examples of herein suitable pressure sensors are well known in the art and the skilled person knows how to select a suitable pressor sensor.

The pressure sensor may be of the type selected from the group consisting of a strain gauge pressure sensor, capacitance pressure transducer, piezoelectric pressure transducer, piezoresistive strain gauge sensor, piezoelectric sensor and piezo resistive bridge sensor. The pressure sensors may be housed in a shell allowing its application to aggressive media. The shell may be constructed form materials including stainless steel, plastic. In one embodiment, the pressure sensor is selected from the group consisting of a strain gauge pressure sensor, a capacitance pressure transducer, a piezoelectric pressure transducer, a piezoresistive strain gauge sensor, a piezoelectric sensor and a piezo resistive bridge sensor. In another embodiment, the pressure sensor detects depth.

Examples of herein suitable accelerometers are well known in the art and the skilled person knows how to select a suitable accelerometer.

Modern commercial accelerometers are often small micro electro-mechanical systems (MEMS) that include an acceleration sensor and an angular velocity sensor (i.e. a gyroscope).

In a preferred embodiment, the position determination unit (1) of item (a) further comprises a gyroscope and the software algorithm comprises further following step:

(iii) sampling data about angular velocity from the gyroscope in the position determination unit (1) of item (a).

Preferably, gyroscope is an inertial gyroscope, such as a MEMS gyroscope.

In order to further refine the positioning of the sensor device - the position determination unit (1) may further comprise e.g. a microphone, a magnetometer or a capacitive sensor.

The position determination unit (1) may further comprise a tilt sensor, an inclinometer or other devices including piezoelectric, piezoresistive, capacitive sensor, or micro electromechanical systems components.

Sensors with digital output are convenient when the data must be transmitted without further noise degradation. All MEMS gyroscopes take advantage of the Coriolis effect. The motion sensor may sense motion along one axis of motion or multiple axes of motion, such as the three orthogonal axes X, Y, and Z. The output can be analog, digital or ratiometric to the supply voltage, or any of various types of pulse modulation. The rate at which the motion sensor communicates and/or stores motion data may vary from approximately 1 hertz (Hz) to approximately 1 kHz. However, any rate may be employed. In one embodiment, the accelerometer is selected from an inertial sensor, such as a MEMS inertial sensor.

Microcontroller unit (3)

Examples of herein suitable microcontroller unit (3) are well known in the art and the skilled person knows how to select a suitable microcontroller unit (3).

Preferably, the microcontroller unit (MCU) acts as a small computer on a single integrated circuit or on a few single integrated circuits. The microcontroller unit (3) may comprise a general purpose programmable processor or controller for executing application programming or instructions related to the device. Furthermore, it can perform operations for configuring and transmitting information as described herein. The microcontroller unit (3) may have one or more central processing units (CPUs) or microprocessors along with RAM for data and calculation, a ROM for boot, memory for program storage and programmable input/output peripherals. As examples, the memory/storage may comprise a computer-readable device, RAM, ROM, DRAM, SDRAM, and/or other storage device(s) and media. The CPU controls the program execution and numerous peripherals for communication with onboard sensors. The software algorithm is part of the program and preferably resides in the memory. The timers are used for timing external event, generating event on periodic basis and for generation of pulse-width modulation (PWM) signals. By way of example, the MCU may comprise a specially configured Application Specific Integrated Circuit (ASIC) or other integrated circuit, a digital signal processor, a controller, a hardwired electronic or logic circuit, a programmable logic device or gate array, a special purpose computer, or the like. In one embodiment, the microcontroller unit (MCU) comprises a microprocessor as means for data processing.

Power management unit (5)

Examples of herein suitable power management units (5) are well known in the art and the skilled person knows how to select a suitable power management unit (5).

The sensor device can be reliant on different power consumption depending on the overall state of the system. The different units of sensor device can be powered down individually in order to save power. The power management unit (5) may be any type of unit capable of storing power over a period of time, such as a rechargeable battery (e.g., Nickel Cadmium or "NiCd", Nickel Metal Hydride or "NiMH", Lithium Ion or "Li Ion", Sealed Lead Acid or "SLA"), a capacitor, a potential-energy-based power storage unit, a chemical-energy-based power storage unit, a kinetic-energy-based power storage unit, or some combination thereof. Reference to the "battery" of the sensor device herein should be understood to refer to any of these types of power storage units. The power management unit (5) may also include embedded sensors and/or processors. For example, some rechargeable batteries (e.g., most modern lithium-ion rechargeable batteries) include sensors and processors that, working together, limit or otherwise control discharging and recharging of the rechargeable battery in ways that help preserve or increase the battery's lifespan, or that help prevent potentially dangerous conditions from forming while the rechargeable battery is discharging or recharging. In one embodiment, the sensor device comprises a power management unit (5). In another embodiment power management unit (5) comprises a battery such as a rechargeable battery.

Process parameter sensor (2)

Examples of herein suitable process parameter sensors (2) are well known in the art and the skilled person knows how to select a suitable process parameter sensor (2) in relation to process parameters of interest (e.g. pH, temperature, level of Oxygen etc.).

The skilled person knows which sensors that may be suitably for monitoring different process parameters in e.g. large vessels during for example fermentation processes or e.g. water purification.

In a preferred embodiment, the process parameter sensor (2) is a sensor that can measure at least one parameter from the group of parameters consisting of: temperature, salts, oxygen, carbon dioxide, a gas, pH, amount of media components such as sugars (e.g. glucose), amino acid and metabolites (e.g. lactate, acetate, ethanol).

Preferably, the process parameter sensor (2) is a sensor that can measure at least one parameter from the group of parameters consisting of: temperature, oxygen and pH.

Preferably, the parameter sensor (2) can measure at least two parameter - such as e.g. temperature and pH.

Other important process parameters could be cell size, cell numbers and cell aggregation. Examples of different sensor that may be used for measuring such process parameters include light sensor, fluorescence sensors, conductivity sensors, optical sensors, temperature sensors, pressure sensors, pH sensors, turbidity sensors, oxygen sensors, carbon dioxide sensors, linear sensor arrays, phased sensor arrays, as well as any other appropriate type and/or arrangement of sensors. Moreover, sensors could also be in the form of cameras and light scatter apparatus. In one embodiment, the process parameter sensor (2) comprises one or more sensors selected from the group consisting of a pH sensor, an oxygen sensor, a refractive index sensor, a cell density sensor, and a temperature sensor.

Communication unit (4)

Examples of herein suitable communication units (4) are well known in the art and the skilled person knows how to select a suitable communication unit (4).

The communication unit (4) is adapted for communicating with a user interface - as understood by the skilled person this is necessary for that the measured process parameters can be received to a user interface (e.g. a computer or screen placed outside the container comprising a liquid medium (e.g. a bioreactor with fermentation liquid medium).

The communication unit (4) may include a transmitter and receiver which can transmit and receive signals respectively, to and from other wireless devices. It also contains one more antennas for use in wireless communications such as multi-input multi-output (MFMO) communications, Bluetooth®, etc. The antennas can include, but are not limited to directional antennas, omnidirectional antennas, monopoles, patch antennas, loop antennas, microstrip antennas, dipoles, and any other antenna(s) suitable for communication transmission/reception.

The communication unit (4) may also optionally contain a security module. This security module can contain information regarding but not limited to, security parameters required to connect the device to an access point or other device or other available network(s), and can include WEP or WPA security access keys, network keys, etc. The WEP security access key is a security password used by Wi-Fi networks. Knowledge of this code will enable a wireless device to exchange information with the access point. The information exchange can occur through encoded messages with the WEP access code often being chosen by the network administrator. WPA is an added security standard that is also used in conjunction with network connectivity with stronger encryption than WEP. In one embodiment the communication is wireless. In another embodiment, the position determination unit (1), the microcontroller unit (3), the power management unit (5), the process parameter sensor (2), and the communication unit (4) are assembled on one printed circuit board (PCB).

Software algorithm as such

The actual mathematics (e.g. integration based mathematics) and specific software language used for making a software algorithm of the first aspect may be seen as based on standard well known mathematics and software language - i.e. based on the teaching herein and the common general knowledge of the skilled person, it is routine work for the skilled person to make a software algorithm of the first aspect of the present invention.

In working example herein is provided a discussion of examples of suitable preferred relevant actual mathematics (e.g. integration based mathematics) and specific software language used for making a software algorithm as described herein.

Software algorithm - step (i) sampling data from the position determination unit (1)

This may be done according to the art - the skilled person knows how to sample data about a parameter of interest - such as e.g. pressure and/or acceleration obtained from the used sensors of the position determination unit (1).

Software algorithm - step (iia) determining the immersion depth and vertical position

As discussed above - step (iia) of the first aspect relates to determining the immersion depth and thereby the vertical position of the sensor device in the liquid medium of the container based on the in step (i) sampled pressure data wherein the pressure is correlated to the depth in the sense that higher pressure relates to increased immersion depth of the sensor device in the liquid medium of the container.
This may be done according to the art - e.g. as discussed in above discussed article by Thiele et al 2010.

Software algorithm - step (iib) determining the horizontal position

As discussed above - step (iib) of the first aspect relates to determining horizontal position of the sensor device in the liquid medium of the container based on the in step (i) sampled accelerometer data, wherein a first acceleration profile is derived from the sensor device contacting the agitator and a second acceleration profile is derived from the sensor device contacting the wall of the container and wherein the first and seconds acceleration profiles are different and the algorithm is capable of identifying this difference and thereby determining when the device contacts the agitator or contacts the wall of the container.
As discussed above, this is a novel essential step of the present invention.
In essence, the present inventors identified that the difference in the first (agitator) acceleration profile and the second (wall) acceleration profile is big enough in order to be measured properly (e.g. in bioreactor) and thereby be used to differentiate between contact/impact of the sensor device and the wall or the agitator by use a of suitable software algorithm.

Software algorithm - sensor device with gyroscope

In a preferred embodiment, the sensor device of first aspect is a sensor device, wherein the position determination unit (1) of item (a) further comprises a gyroscope and the software algorithm comprises further following step:

(iii) sampling data about angular velocity from the gyroscope in the position determination unit (1) of item (a); and
(iv): determining position of the sensor device via the microcontroller unit (3) of item (b) of the first aspect, wherein the in step (i) of the first aspect sampled accelerometer data and the in step (iii) sampled gyroscope data continuously calculate the position, orientation, and velocity (direction and speed of movement) of the moving sensor device within the liquid media of the container by a process of calculating the sensor device current position by using a previously determined position and the previously determined position is when the device contacts the agitator and when the device contacts the wall of the container as determined in step (iib) of the first aspect.

As understood by the skilled person, the sampled data of step (iv) are used to continuously calculate the position, orientation, and velocity (direction and speed of movement) of the moving sensor device.
As known the art - in e.g. navigation, dead reckoning or dead-reckoning (also ded for deduced reckoning or DR) is the process of calculating one's current position by using a previously determined position - i.e. navigation dead reckoning known methods may be seen as suitable herein relevant methods for step (iv) above.

Use of the sensor device for measuring process parameters in a container:

As discussed above, a second aspect of the invention relates to use of the sensor device of first aspect and relevant embodiments thereof for measuring position and process parameters in a container comprising a liquid medium, a wall and an agitator.
As known in the art - an example of a suitable agitator is an impeller.
Accordingly, in an embodiment of the present invention the agitator is an impeller.
Preferably, the container (e.g. a bioreactor) is a container comprising at least 100 L of liquid medium, such as at least 1000 L of liquid medium.

The liquid medium of the container may preferably comprise a polypeptide of interest, e.g. a protein of interest such as e.g. an enzyme of interest.

Preferably, the container is a bioreactor.

When the container is a bioreactor it is preferred that the liquid medium is a fermentation liquid medium, where it may be preferred that the bioreactor is used for growth of microorganism such as e.g. bacterial, fungal, yeasts or mammalian cell.

Preferably, the growth of microorganism is for recombinant production of a polypeptide of interest, e.g. a protein of interest such as e.g. an enzyme of interest.

In the preferred embodiment, the container is cylindrical.

An advantage of use of a cylindrical container is that the composition (e.g. amount of $O_2$ and pH level) is similar at one immersion depth (vertical position) and the same distance from e.g. the wall (horizontal position) of the container.

As used herein the term "bioreactor" refers to any device or system that supports a biologically active environment. In one case but not limited to, a bioreactor is a container or vessel in which is carried out a chemical process which involves organisms or biochemically active substances derived from such organisms. This process can either be aerobic or anaerobic. Bioreactors are commonly cylindrical, ranging in size from some litres to cubic meters, and are often made of stainless steel but could also be made of other materials such as disposable materials.

A bioreactor may also refer to a device or system meant to grow cells or tissues in the context of cell culture. On the basis of mode of operation, a bioreactor may be classified as batch, fed-batch or continuous (e.g. continuous stirred-tank reactor model). An example of a bioreactor is the chemostat. The bioreactor may be equipped with one or more inlets for supplying new fresh or concentrated medium to the cells, and with one or more outlets for harvesting product or emptying the bioreactor. Additionally, the bioreactor may be equipped with at least one outlet constructed in such a way that a separation device can be attached to the bioreactor. Typically the bioreactor's environmental conditions like gas (i.e., air, oxygen, nitrogen, carbon dioxide) flow rates, temperature, pH and dissolved oxygen levels, and agitation speed/circulation rate can be closely monitored and controlled.

A method for determining the position of the sensor device:

As discussed above, a third aspect of the invention relates to a method for determining the position of the sensor device of the first aspect and any relevant embodiments thereof in a container comprising a liquid medium, a wall and an agitator comprising the steps of:

(i) sampling data about pressure and acceleration from the position determination unit (1) of item (a); and

(iia) determining the immersion depth and thereby the vertical position of the sensor device in the liquid medium of the container based on the in step (i) sampled pressure data wherein the pressure is correlated to the depth in the sense that higher pressure relates to increased immersion depth of the sensor device in the liquid medium of the container; and

(iib): determining horizontal position of the sensor device in the liquid medium of the container based on the in step (i) sampled accelerometer data, wherein a first acceleration profile is derived from the sensor device contacting the agitator and a second acceleration profile is derived from the sensor device contacting the wall of the container and wherein the first and seconds acceleration profiles are different and the algorithm is capable of identifying this difference and thereby determining when the device contacts the agitator or contacts the wall of the container.

Preferred embodiments of the individual steps are described above.

## EXAMPLES

### EXAMPLE 1: *Software algorithm*

The actual mathematics (e.g. integration based mathematics) and specific software language used for making a software algorithm of the first aspect may be seen as based on standard well known mathematics and software language - i.e. based on the teaching herein and the common general knowledge of the skilled person, it is routine work for the skilled person to make a software algorithm of the first aspect of the present invention.

Below is provided a discussion of examples of suitable preferred relevant actual mathematics (e.g. integration based mathematics) and specific software language used for making a software algorithm as described herein.

The developed positioning Algorithm has roots in literature from pedestrian inertial navigation. Raw output data from the position determination unit (1) (acceleration and angular velocity in 3 axis) over a time interval is used to obtain the position of the particle. The acceleration [m/s/s] measured by the accelerometer is integrated twice to obtain the distance travelled [m] during a period, while the gyroscope which measures angular velocity [rad/sec], needs to be integrated once to obtain the rotation [rad]. Before integrating the raw data, the following operations have to be performed.

1. Gravity compensation is required (subtraction of gravitational acceleration from accelerometer output). Before the subtraction is done, the coordinates of the IMU (body coordinates) must be facing in the same direction as the "world frame" or "navigation frame". This requires rotation operations which can be carried out by the use quaternions. Quaternions are a convenient way of representing rotations and can easily be converted to rotation matrices or Euler's angles [1]. This first step is represented below:

$$a_n = R_n f_n - g$$

Where $a_n$ is the acceleration in world frame, $R_n$ is a rotation matrix, $f_n$ is the measured acceleration (specific force) and g is the gravitational acceleration vector, g = $[0\,0\,9.81]^T$. $n$ refers to the current state.

2. The acceleration in world frame, $a_n$, can now be integrated over the sample period twice to obtain the position.

3. MEMS sensors are very sensitive and errors from double integration accumulate rapidly. These errors can be restrained by using independent inputs describing the same variables. An example of this is the zero velocity update (ZUPT) [2]. ZUPT exploits moments with none or little motion to correct the calculated velocity.

Furthermore, there are several ways to obtain better estimates of the position. In this algorithm, the Kalman filter [3] based on the states and models by [4], is used to obtain the best estimates that includes noise and bias of the measurements.
The filter uses the basic mechanical equations of motions,

$$p_n = p_{n-1} + v_{n-1} T_s + \frac{1}{2} a_n T_s^2, (1)$$

$$v_n = v_{n-1} + a_n T_s, \qquad (2)$$

Where p is the position, $v$, the velocity, a, the acceleration and $T_s$ is the sample period. The angles are updated by:

$$q_n = \Omega(\omega_n T_s) q_{n-1},$$

where $q_n$ and $q_{n-1}$ are quaternions describing the rotation at the current state and previous state, respectively. $\omega_n$ is a vector containing the input angles obtained by gyroscope measurements, and $\Omega$ is a quaternion update matrix.
The state vector used in the model are:

$$x_n = \begin{bmatrix} p_{x.n} & p_{y,n} & p_{z,n} & v_{x,n} & v_{y,n} & v_{z,n} & \theta_{r,n} & \theta_{p,n} & \theta_{y,n} \end{bmatrix}^T$$

Where p and $v$ are calculated from equation 1 and 2 and $\theta_r$, $\theta_p$ and $\theta_y$ are Euler's angles converted from quaternions, roll, pitch and yaw, respectively.
The acceleration can be assumed constant in short sampling period or a linear approximation can be used.
The ZUPT is applied by using pseudo measurements of the velocity in the observation matrix. The pseudo measurements are zero with some noise added from the acceleration measurement. In addition, the z-coordinate obtained by the pressure sensor is added to this matrix in order to obtain better estimates of $p_z$. The measurement vector, z, becomes:

$$z = \begin{bmatrix} p_z & v_{x,pseudo} & v_{y,pseudo} & v_{z,pseudo} \end{bmatrix}^T$$

The pseudo measurements of velocity are only applied in the period with none or negligible movement, while the correction for $p_z$ is applied at every state. As an alternative, a z velocity component can be calculated from the pressure measurements if the absolute depth (density) is not known, but the density can be assumed constant. If there is no motion at all (i.e. no movement and no rotation), then it is possible to correct the roll and pitch as well, due to the direction of the gravitational acceleration.

The algorithm also resets the position to $p = [0\ 0\ p_z]$ on impact with the wall of the fermentation tank, which results in trajectories as output, rather than absolute position.

Herein relevant references include:

Diebel, J. (2006). Representing Attitude: Euler Angles, Unit Quaternions, and Rotation Vectors. Section 6.

Abdulrahim, K., Moore, T., Hide, C., Hill, C. (2014). Understanding the Performance of Zero Velocity Updates in MEMS-based Pedestrian Navigation. International Journal of Advancements in Technology, 4: 53-60.

Welch, G., Bishop, G. (2001). An introduction to the Kalman Filter. University of North Carolina at Chapel Hill. Department of Computer Science.

Nilsson, J., Gupta, A. K., Handel, P. (2014) Foot-mounted inertial navigation made easy. International Conference on Indoor Positioning and Indoor Navigation

Colomar, D. S., Nilsson, J., Handel, P. (2012). Smoothing for ZUPT-aided INSs. International Conference on Indoor Positioning and Indoor Navigation

**EXAMPLE 2:** *Test of sensor device in a bioreactor*

The sensor device was the sensor device as shown in Figure 1a comprising (using numbering of block diagram of Figure 1b):

a house (10), wherein the house (10) comprises:

(a) a position determination unit (1) comprising an accelerometer and a pressure sensor,
(b) a microcontroller unit (3) comprising a software algorithm configured for determining position of the sensor device,
(c) a power management unit (5),
(d) a process parameter sensor (2), and
(e) a communication unit (4) adapted for communicating with a user interface.

As known in the art - relevant electronic components (e.g. sensors) are commercially available - e.g. at the website of the company Farnell (www.farnell.com).

In relation to (a) - the accelerometer was a commercially available ADXL375 from Analog devices and the pressure sensor was a commercially available MSS5803-05 from TE Connectivity.

In relation to (b) - the microcontroller unit (3) was a microcontroller unit comprising a standard MCU with RAM for data and calculation, a ROM for boot, flash for program storage and tables, a CPU for program execution and numerous peripherals for communication with onboard sensors etc. The software algorithm is part of the program and resides in the flash. The timers are used for timing external event, generating event on periodic basis and for generation of PWM signals.

In relation to (c) - the power management unit (5) was a power management unit comprising battery and several voltage regulators. Generally speaking, the power management unit may be different power units depending on the overall state of the system. Blocks can be powered down individually in order to save power and only power needed sensors, internal devices etc.

In relation to (d) - the process parameter sensors (2) were two sensors - one capable of measuring temperature and one capable of measuring pH. The temperature sensor was included in the above described commercially available pressure sensor and the pH sensor was a sensor based on pH meter ISFET technology.

In relation to (e) - the communication unit (4) adapted for communicating with a user interface a unit based on wireless communication. The unit contained a radio transceiver and an antenna for wireless communication with the outside world.

The sensor device was tested in a 1 $m^3$ bioreactor with a diameter of 0.92 m. The bioreactor was filled with 0.66 $m^3$ of pure water to reach a liquid height of 1 m. The liquid was agitated by two Rushton turbines (i.e. impellers) with a clearance of approximately 0.35 m, a diameter of 0.3 m and a rotation speed of 175 rpm.

The software algorithm in (b) of the sensor device was an algorithm capable of:

(iia) determining the immersion depth and thereby the vertical position of the sensor device in the liquid medium of the bioreactor based on the sampled pressure data wherein the pressure is correlated to the depth in the sense that higher pressure relates to increased immersion depth of the sensor device in the liquid medium of the container; and
(iib): determining horizontal position of the sensor device in the liquid medium of the bioreactor based on the sampled accelerometer data, wherein a first acceleration profile is derived from the sensor device contacting the agitator/impeller and a second acceleration profile is derived from the sensor device contacting the wall of the container and wherein the first and seconds acceleration profiles are different and the algorithm is capable of identifying this difference and thereby determining when the device contacts the agitator or contacts the wall of the container.

Herein relevant details of the software algorithm are discussed below.

The software algorithm was configured to measure acceleration profiles of impacts at 3200 Hz and the pressure at 100 Hz.

The shock accelerometer profiles were processed to differentiate between impacts with the wall and the impellers. The differentiation was based on a threshold of in the weighted mean, p, given by:

$$p = \frac{\alpha a + \beta b + \gamma c + \delta d}{\alpha + \beta + \gamma + \delta}.$$

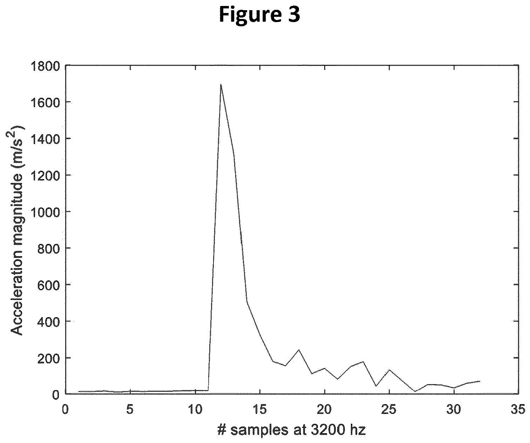

The value of p is calculated by a fuzzy logic algorithm in which $\alpha$, $\beta$, $\gamma$ and $\delta$ are weight parameters and a, b, c and d are functions of the shape of the acceleration profile. An example of a measured impact profile is shown in figure 3.

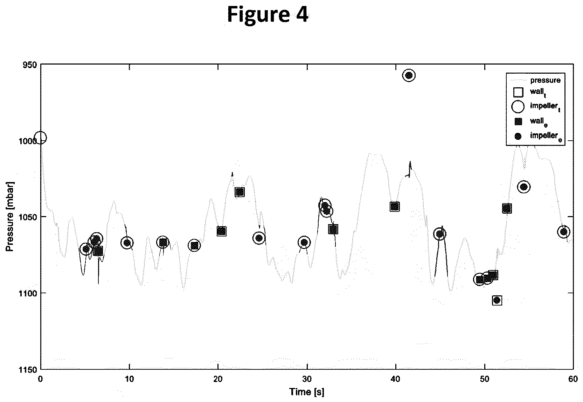

Figure 4 shows a comparison of impacts with wall and impellers which are theoretically determined by the algorithm and the corresponding impacts which are determined by visual inspection. A correct match of geometric figures indicates a correct determination. The grey line is the measured pressure which can be used to calculate the immersion depth (i.e. vertical position). It can be seen that the algorithm has a fairly high success rate, which can be further optimized by tweaking the algorithm.

In conclusion - the algorithm of the sensor device was able to determine the horizontal position of the sensor device in the liquid medium of the bioreactor based on the sampled accelerometer data, wherein a first acceleration profile was derived from the sensor device contacting the agitator and a second acceleration profile was derived from the sensor device contacting the wall of the container and wherein the first and seconds acceleration profiles were different and the algorithm was capable of identifying this difference and thereby determining when the device contacts the agitator/impeller or contacts the wall of the container.

As discussed above, the sensor device comprised two process parameter sensors (2) - one capable of measuring temperature and one capable of measuring pH.

These two parameters were continuously measured and correlated with position of the sensor - i.e. specific temperature/pH parameters were obtained for specific positions of the sensor device within the liquid medium of the bioreactor. It is evident that such information is very useful for optimization of e.g. fermentation/growth conditions of the bioreactor.

**REFERENCE LIST**

1: Thiele et al., "Autonomous sensor particle for parameter tracking in large vessels", Meas. Sci. Technol. vol. 21 (2010) page 085201 (8pp).

2: Reinecke et al., "Macro-mixing characterisation of a stirred model fermenter of non-Newtonian liquid by flow following sensor particles and ERT. Chemical Engineering Research and Design vol. 118, (2016-12-09) pages 1-11.

**Claims**

1. A sensor device comprising a house (10), wherein the house (10) comprises:

   (a) a position determination unit (1) comprising an accelerometer and a pressure sensor,
   (b) a microcontroller unit (3) comprising a software algorithm configured for determining position of the sensor device,
   (c) a power management unit (5),
   (d) a process parameter sensor (2), and
   (e) a communication unit (4) adapted for communicating with a user interface; and

   wherein the software algorithm in (b) can determine the position of the sensor device in a container comprising a liquid medium, a wall and an agitator wherein the algorithm is an algorithm comprising the following steps:

   (i) sampling data about pressure and acceleration from the position determination unit (1) of item (a); and
   (iia) determining the immersion depth and thereby the vertical position of the sensor device in the liquid medium of the container based on the in step (i) sampled pressure data wherein the pressure is correlated to the depth in the sense that higher pressure relates to increased immersion depth of the sensor device in the liquid medium of the container; and

(iib): determining horizontal position of the sensor device in the liquid medium of the container based on the in step (i) sampled accelerometer data, wherein a first acceleration profile is derived from the sensor device contacting the agitator and a second acceleration profile is derived from the sensor device contacting the wall of the container and wherein the first and seconds acceleration profiles are different and the algorithm is capable of identifying this difference and thereby determining when the device contacts the agitator or contacts the wall of the container.

2. The sensor device of claim 1, wherein the position determination unit (1) of item (a) further comprises a gyroscope and the software algorithm comprises further following steps:

(iii) sampling data about angular velocity from the gyroscope in the position determination unit (1) of item (a); and

(iv): determining position of the sensor device via the microcontroller unit (3) of item (b) of claim 1, wherein the in step (i) of claim 1 sampled accelerometer data and the in step (iii) sampled gyroscope data continuously calculate the position, orientation, and velocity (direction and speed of movement) of the moving sensor device within the liquid media of the container by a process of calculating the sensor device current position by using a previously determined position and the previously determined position is when the device contacts the agitator and when the device contacts the wall of the container as determined in step (iib) of claim 1.

3. The sensor device of any one of the preceding claims, wherein the house (10) is in a two-part form.

4. The sensor device of any one of the preceding claims, wherein the house (10) is made of a bio compatible product including metal, wood, bamboo and plastic such as organic thermoplastic polymer plastic including polyether ether ketone (PEEK) and polyaryl ether ketone (PAEK).

5. The sensor device of any one of the preceding claims, wherein the house (10) comprises a data storage unit and/or the weight of the sensor device is 1 g to 500 g.

6. The sensor device of any one of the preceding claims, wherein the process parameter sensor (2) comprises one or more sensors selected from the group consisting of a pH sensor, an oxygen sensor, a refractive index sensor, a cell density sensor, and a temperature sensor.

7. The sensor device of any one of the preceding claims, wherein the sensor device is shaped as a sphere and/or the size of the sensor device is a size with a diameter from 1 mm to 100 cm.

8. The sensor device of any one of the preceding claims, wherein the pressure sensor is selected from the group consisting of a strain gauge pressure sensor, capacitance pressure transducer, piezoelectric pressure transducer, piezoresistive strain gauge sensor, piezoelectric sensor and piezo resistive bridge sensor.

9. The sensor device of any one of the preceding claims, wherein the accelerometer is selected from an inertial sensor, such as a MEMS inertial sensor.

10. The sensor device of any one of the preceding claims, wherein the power management unit (5) comprises a rechargeable battery and/or wherein the communication unit (4) is wireless.

11. The sensor device of any one of the preceding claims, wherein the software algorithm of claim 1 is part of a program that resides in the memory of the microcontroller unit (3) and which is executed by a central processing unit (CPU) of the microcontroller unit (3).

12. Use of the sensor device of any one of claims 1-11 for measuring position and process parameters in a container comprising a liquid medium, a wall and an agitator.

13. The use of claim 12, wherein the agitator is an impeller and the liquid medium of the container comprises a polypeptide of interest.

14. The use of any one of claims 12-13, wherein the container is a bioreactor and the bioreactor comprises at least 100 L of liquid medium and the liquid medium is a fermentation liquid medium and the bioreactor is used for growth of microorganisms and the growth of microorganisms is for recombinant production of a polypeptide of interest.

15. The use of any one of claims 12-14, wherein the container is cylindrical.

16. A method for determining the position of the sensor device of any one of claims 1-11 in a container comprising a liquid medium, a wall and an agitator comprising the steps of:

(i) sampling data about pressure and acceleration from the position determination unit (1) of item (a); and
(iia) determining the immersion depth and thereby the vertical position of the sensor device in the liquid medium of the container based on the in step (i) sampled pressure data wherein the pressure is correlated to the depth in the sense that higher pressure relates to increased immersion depth of the sensor device in the liquid medium of the container; and
(iib): determining horizontal position of the sensor device in the liquid medium of the container based on the in step (i) sampled accelerometer data, wherein a first acceleration profile is derived from the sensor device contacting the agitator and a second acceleration profile is derived from the sensor device contacting the wall of the container and wherein the first and seconds acceleration profiles are different and the algorithm is capable of identifying this difference and thereby determining when the device contacts the agitator or contacts the wall of the container.

**Patentansprüche**

1. Eine Sensorvorrichtung umfassend ein Gehäuse (10), wobei das Gehäuse (10) folgendes umfasst:

(a) eine Positionsfeststellungseinheit (1) umfassend einen Beschleunigungsmesser und einen Drucksensor,
(b) eine Mikrokontrollereinheit (3) umfassend einen Softwarealgorithmus, der zur Feststellung der Position der Sensorvorrichtung konfiguriert ist,
(c) eine Leistungsmanagementeinheit (5),
(d) einen Verfahrensparametersensor (2), und
(e) eine Verbindungseinheit (4), die zur Verbindung mit einer Benutzerschnittstelle angepasst ist; und

wobei der Softwarealgorithmus von (b) die Position der Sensorvorrichtung in einem Behälter umfassend ein flüssiges Medium, eine Wand und einen Rührer feststellen kann, wobei der Algorithmus ein Algorithmus ist, der folgende Schritte umfasst:

(i) die Abtastung von Daten von Druck und Beschleunigung aus der Positionsfeststellungseinheit (1) des Bestandteils (a); und
(iia) die Feststellung von der Eintauchtiefe und somit von der vertikalen Position der Sensorvorrichtung im flüssigen Medium des Behälters beruhend auf den in Schritt (i) abgetasteten Druckdaten, wobei der Druck insofern in Zusammenhang mit der Tiefe steht, als ein höherer Druck mit einer größeren Eintauchtiefe der Sensorvorrichtung in dem flüssigen Medium des Behälters verbunden ist; und
(iib): die Feststellung von der horizontalen Position der Sensorvorrichtung im flüssigen Medium des Behälters beruhend auf den in Schritt (i) abgetasteten Beschleunigungsmesserdaten, wobei ein erstes Beschleunigungsprofil aus einem Kontakt der Sensorvorrichtung mit dem Rührer abgeleitet ist und ein zweites Beschleunigungsprofil aus einem Kontakt der Sensorvorrichtung mit der Behälterwand abgeleitet ist, und wobei das erste und das zweite Beschleunigungsprofil abweichend sind und der Algorithmus diese Abweichung identifizieren kann und somit feststellen kann, wann die Vorrichtung in Kontakt mit dem Rührer oder in Kontakt mit der Behälterwand kommt.

2. Die Sensorvorrichtung des Anspruchs 1, wobei die Positionsfeststellungseinheit (1) des Bestandteils (a) weiterhin ein Gyroskop umfasst und der Softwarealgorithmus weiterhin folgende Schritte umfasst:

(iii) die Abtastung von Drehgeschwindigkeitsdaten aus dem Gyroskop in der Positionsfeststellungseinheit (1) des Bestandteils (a); und
(iv): die Feststellung der Position der Sensorvorrichtung durch die Mikrokontrollereinheit (3) des Bestandteils (b) des Anspruchs 1, wobei die in Schritt (i) des Anspruchs 1 abgetasteten Beschleunigungsmesserdaten und die in Schritt (iii) abgetasteten Gyroskopdaten die Position, Ausrichtung, und Geschwindigkeit (Richtung und Geschwindigkeitsbetrag der Bewegung) der sich bewegenden Sensorvorrichtung in den flüssigen Medien des Behälters mittels eines Verfahrens kontinuierlich berechnen, in dem die momentane Position der Sensorvorrichtung mittels einer vorher festgestellten Position berechnet wird und wobei die vorher festgestellte Position die Zeit ist, zu der die Vorrichtung in Kontakt mit dem Rührer kommt und die Zeit ist, zu der die Vorrichtung in Kontakt mit der Behälterwand kommt, wie sie in Schritt (iib) des Anspruchs 1 festgestellt sind.

3. Die Sensorvorrichtung von einem der vorhergehenden Ansprüche, wobei das Gehäuse (10) zweiteilig ist.

4. Die Sensorvorrichtung von einem der vorhergehenden Ansprüche, wobei das Gehäuse (10) aus einem biokompatiblen Produkt einschließlich Metall, Holz, Bambus und Kunststoff, wie z.B. einem organischen thermoplastischen polymeren Kunststoff einschließlich Polyetheretherketon (PEEK) und Polyaryletherketon (PAEK) besteht.

5. Die Sensorvorrichtung von einem der vorhergehenden Ansprüche, wobei das Gehäuse (10) eine Datenspeichereinheit umfasst und/oder das Gewicht der Sensorvorrichtung von 1 g bis 500 g reicht.

6. Die Sensorvorrichtung von einem der vorhergehenden Ansprüche, wobei der Verfahrensparametersensor (2) einen oder mehrere Sensoren umfasst, die ausgewählt aus der Gruppe bestehend aus einem pH-Sensor, einem Sauerstoffsensor, einem Brechungsindexsensor, einem Zelldichtesensor und einem Temperatursensor ist.

7. Die Sensorvorrichtung von einem der vorhergehenden Ansprüche, wobei die Sensorvorrichtung sphärisch ist und/oder die Größe der Sensorvorrichtung eine Größe mit einem Durchmesser von 1 mm bis 100 cm ist.

8. Die Sensorvorrichtung von einem der vorhergehenden Ansprüche, wobei der Drucksensor ausgewählt ist aus der Gruppe bestehend aus einem DMS-Drucksensor, einem kapazitiven Druckwandler, einem piezoelektrischen Druckwandler, einem piezoresistiven DMS-Sensor, einem piezoelektrischen Sensor und einem piezoresistiven Brückensensor.

9. Die Sensorvorrichtung von einem der vorhergehenden Ansprüche, wobei der Beschleunigungsmesser ausgewählt aus einem Trägheitssensor, wie z.B. einem MEMS-Trägheitssensor, ist.

10. Die Sensorvorrichtung von einem der vorhergehenden Ansprüche, wobei die Leistungsmanagementeinheit (5) eine wiederaufladbare Batterie umfasst und/oder wobei die Verbindungseinheit (4) drahtlos ist.

11. Die Sensorvorrichtung von einem der vorhergehenden Ansprüche, wobei der Softwarealgorithmus des Anspruchs 1 ein Teil von einem Programm ist, das in dem Speicher der Mikrokontrollereinheit (3) enthalten ist und welches durch eine zentrale Verarbeitungseinheit (CPU) der Mikrokontrollereinheit (3) ausgeführt wird.

12. Verwendung von der Sensorvorrichtung von einem der Ansprüche 1-11 zur Messung der Position und von Verfahrensparametern in einem Behälter umfassend ein flüssiges Medium, eine Wand und einen Rührer.

13. Die Verwendung des Anspruchs 12, wobei der Rührer ein Laufrad ist und das flüssige Medium des Behälters ein Polypeptid von Interesse umfasst.

14. Die Verwendung von einem der Ansprüche 12-13, wobei der Behälter ein Bioreaktor ist und der Bioreaktor mindestens 100 L von flüssigem Medium enthält und das flüssige Medium ein flüssiges Gärungsmedium ist und der Bioreaktor zum Wachstum von Mikroorganismen verwendet wird und das Wachstum der Mikroorganismen zur rekombinanten Herstellung von einem Polypeptid von Interesse ist.

15. Die Verwendung von einem der Ansprüche 12-14, wobei der Behälter zylindrisch ist.

16. Ein Verfahren zur Feststellung der Position der Sensorvorrichtung von einem der Ansprüche 1-11 in einem Behälter umfassend ein flüssiges Medium, eine Wand und einen Rührer umfassend folgende Schritte:

(i) die Abtastung von Daten von Druck und Beschleunigung aus der Positionsfeststellungseinheit (1) des Bestandteils (a); und
(iia) die Feststellung von der Eintauchtiefe und somit von der vertikalen Position der Sensorvorrichtung im flüssigen Medium des Behälters beruhend auf den in Schritt (i) abgetasteten Druckdaten, wobei der Druck insofern in Zusammenhang mit der Tiefe steht, als ein höherer Druck mit einer größeren Eintauchtiefe der Sensorvorrichtung in dem flüssigen Medium des Behälters verbunden ist; und

(iib): die Feststellung von der horizontalen Position der Sensorvorrichtung im flüssigen Medium des Behälters beruhend auf den in Schritt (i) abgetasteten Beschleunigungsmesserdaten, wobei ein erstes Beschleunigungsprofil aus einem Kontakt der Sensorvorrichtung mit dem Rührer abgeleitet ist und ein zweites Beschleunigungsprofil aus einem Kontakt der Sensorvorrichtung mit der Behälterwand abgeleitet ist, und wobei das erste und das zweite Beschleunigsprofil abweichend sind und der Algorithmus diese Abweichung identifizieren kann und somit feststellen kann, wann die Vorrichtung in Kontakt mit dem Rührer oder in Kontakt mit der Behälterwand kommt.

## Revendications

1. Un dispositif capteur comprenant un boîtier (10), dans lequel le boîtier (10) comprend :

     (a) une unité de détermination de position (1) comprenant un accéléromètre et un capteur de pression,
     (b) une unité de microcontrôleur (3) comprenant un algorithme de software configuré pour déterminer la position du dispositif capteur,
     (c) une unité de contrôle de puissance (5),
     (d) un capteur de paramètres de processus (2), et
     (e) une unité de communication (4) adaptée pour la communication avec une interface d'utilisateur ; et

     dans lequel l'algorithme de software de (b) peut déterminer la position du dispositif capteur dans un conteneur comprenant un milieu liquide, une paroi et un agitateur, dans lequel l'algorithme est un algorithme comprenant les étapes suivantes :

     (i) échantillonner des données sur la pression et l'accélération obtenues de l'unité de détermination de position (1) de l'élément (a) ; et
     (iia) déterminer la profondeur d'immersion et donc la position verticale du dispositif capteur dans le milieu liquide du conteneur sur la base des données de pression échantillonnées dans l'étape (i), dans lequel la pression est corrélée à la profondeur dans le sens qu'une pression plus haute est liée à une profondeur d'immersion augmentée du dispositif capteur dans le milieu liquide du conteneur ; et
     (iib): déterminer la position horizontale du dispositif capteur dans le milieu liquide du conteneur sur la base des données d'accéléromètre échantillonnées dans l'étape (i), dans lequel un premier profile d'accélération est dérivé du dispositif capteur en contact avec l'agitateur et un second profile d'accélération est dérivé du dispositif capteur en contact avec la paroi du conteneur et dans lequel les profils d'accélération premier et second sont différents et l'algorithme est capable d'identifier cette différence et déterminer ainsi le moment où le dispositif vient en contact avec l'agitateur ou vient en contact avec la paroi du conteneur.

2. Le dispositif capteur de la revendication 1, dans lequel l'unité de détermination de position (1) de l'élément (a) comprend en outre un gyroscope et l'algorithme de software comprend les étapes additionnelles suivantes :

     (iii) échantillonner des données sur la vitesse angulaire provenant du gyroscope dans l'unité de détermination de position (1) de l'élément (a) ; et
     (iv): déterminer la position du dispositif capteur à travers l'unité de microcontrôleur (3) de l'élément (b) de la revendication 1, dans lequel les données d'accéléromètre échantillonnées dans l'étape (i) de la revendication 1 et les données du gyroscope échantillonnées dans l'étape (iii) calculent de manière continuelle la position, l'orientation, et la vélocité (direction et vitesse de mouvement) du dispositif capteur en mouvement dans les milieux liquides du conteneur moyennant un processus de calcul de la position instantanée du dispositif capteur en utilisant une position déterminée auparavant et la position déterminée auparavant est le moment où le dispositif vient en contact avec l'agitateur et le moment où le dispositif vient en contact avec la paroi du conteneur tels que déterminés dans l'étape (iib) de la revendication 1.

3. Le dispositif capteur de l'une quelconque des revendications précédentes, dans lequel le boîtier (10) a une forme en deux parties.

4. Le dispositif capteur de l'une quelconque des revendications précédentes, dans lequel le boîtier (10) est réalisé en un produit biocompatible incluant du métal, du bois, du bambou et du plastique, tel qu'un plastique polymère thermoplastique organique incluant de la polyétheréthercétone (PEEK) et de la polyaryléthercétone (PAEK).

5. Le dispositif capteur de l'une quelconque des revendications précédentes, dans lequel le boîtier (10) comprend une unité de stockage de données et/ou le poids du dispositif capteur va de 1 g à 500 g.

6. Le dispositif capteur de l'une quelconque des revendications précédentes, dans lequel le capteur de paramètres de processus (2) comprend un ou plus capteurs choisis dans le groupe constitué d'un capteur de pH, un capteur d'oxygène, un capteur d'indice de réfraction, un capteur de densité cellulaire, et un capteur de température.

7. Le dispositif capteur de l'une quelconque des revendications précédentes, dans lequel le dispositif capteur a la forme d'une sphère et/ou la taille du dispositif capteur est une taille avec un diamètre allant de 1 mm à 100 cm.

8. Le dispositif capteur de l'une quelconque des revendications précédentes, dans lequel le capteur de pression est choisi dans le groupe constitué d'un capteur de pression à jauges de déformation, un transducteur capacitif de pression, un transducteur piézoélectrique de pression, un capteur à jauges de déformation piézorésistif, un capteur piézoélectrique et un capteur en pont piézorésistif.

9. Le dispositif capteur de l'une quelconque des revendications précédentes, dans lequel l'accéléromètre est choisi parmi un capteur inertiel, tel qu'un capteur inertiel MEMS.

10. Le dispositif capteur de l'une quelconque des revendications précédentes, dans lequel l'unité de contrôle de puissance (5) comprend une batterie rechargeable et/ou dans lequel l'unité de communication (4) est sans fil.

11. Le dispositif capteur de l'une quelconque des revendications précédentes, dans lequel l'algorithme de software de la revendication 1 fait partie d'un programme résidant dans la mémoire de l'unité de microcontrôleur (3) et qui est exécuté par une unité centrale de traitement (UCT) de l'unité de microcontrôleur (3).

12. Utilisation du dispositif capteur de l'une quelconque des revendications 1-11 pour mesurer la position et des paramètres de processus d'un conteneur comprenant un milieu liquide, une paroi et un agitateur.

13. L'utilisation de la revendication 12, dans laquelle l'agitateur est un impulseur et le milieu liquide du conteneur comprend un polypeptide d'intérêt.

14. L'utilisation de l'une quelconque des revendications 12-13, dans laquelle le conteneur est un bioréacteur et le bioréacteur comprend au moins 100 L de milieu liquide et le milieu liquide est un milieu liquide de fermentation et le bioréacteur est utilisé pour la croissance de microorganismes et la croissance de microorganismes est pour la production recombinante d'un polypeptide d'intérêt.

15. L'utilisation de l'une quelconque des revendications 12-14, dans laquelle le conteneur est cylindrique.

16. Un procédé de détermination de la position du dispositif capteur de l'une quelconque des revendications 1-11 dans un conteneur comprenant un milieu liquide, une paroi et un agitateur comprenant les étapes suivantes :

(i) échantillonner des données sur la pression et l'accélération obtenues de l'unité de détermination de position (1) de l'élément (a) ; et

(iia) déterminer la profondeur d'immersion et donc la position verticale du dispositif capteur dans le milieu liquide du conteneur sur la base des données de pression échantillonnées dans l'étape (i), dans lequel la pression est corrélée à la profondeur dans le sens qu'une pression plus haute est liée à une profondeur d'immersion augmentée du dispositif capteur dans le milieu liquide du conteneur ; et

(iib): déterminer la position horizontale du dispositif capteur dans le milieu liquide du conteneur sur la base des données d'accéléromètre échantillonnées dans l'étape (i), dans lequel un premier profile d'accélération est dérivé du dispositif capteur en contact avec l'agitateur et un second profile d'accélération est dérivé du dispositif capteur en contact avec la paroi du conteneur et dans lequel les profils d'accélération premier et second sont différents et l'algorithme est capable d'identifier cette différence et déterminer ainsi le moment où le dispositif vient en contact avec l'agitateur ou vient en contact avec la paroi du conteneur.

# Figure 1a

# Figure 1b

House 10

# Figure 2

# Figure 3

# Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DIEBEL, J.** *Representing Attitude: Euler Angles, Unit Quaternions, and Rotation Vectors,* 2006
- **ABDULRAHIM, K. ; MOORE, T. ; HIDE, C. ; HILL, C.** Understanding the Performance of Zero Velocity Updates in MEMS-based Pedestrian Navigation. *International Journal of Advancements in Technology,* 2014, vol. 4, 53-60
- **WELCH, G. ; BISHOP, G.** An introduction to the Kalman Filter. University of North Carolina at Chapel Hill. Department of Computer Science, 2001
- **NILSSON, J. ; GUPTA, A. K. ; HANDEL, P.** Foot-mounted inertial navigation made easy. *International Conference on Indoor Positioning and Indoor Navigation,* 2014
- **COLOMAR, D. S. ; NILSSON, J. ; HANDEL, P.** Smoothing for ZUPT-aided INSs. *International Conference on Indoor Positioning and Indoor Navigation,* 2012
- **THIELE et al.** Autonomous sensor particle for parameter tracking in large vessels. *Meas. Sci. Technol.,* 2010, vol. 21, 085201
- **REINECKE et al.** Macro-mixing characterisation of a stirred model fermenter of non-Newtonian liquid by flow following sensor particles and ERT. *Chemical Engineering Research and Design,* 09 December 2016, vol. 118, 1-11